# EUROPEAN PATENT APPLICATION

(11) **EP 3 552 544 A1**
(43) Date of publication of application: **16.10.2019**
(21) Application number: 18167049.8
(22) Date of filing: 12.04.2018
(51) Int. Cl.: A61B 5/055

(54) **MRI COMPATIBLE FIXATION FRAME**

(71) Applicant: Medical Intelligence Medizintechnik GmbH, 86830 Schwabmünchen (DE)
(72) Inventor: Rui, Liu, 86153 Augsburg (DE)
(74) Representative: Maiwald Patent- und Rechtsanwaltsgesellschaft mbH

(57) **Abstract**

MRI compatible frame for mouthpiece-supported patient positioning for use in a high magnetic field environment comprising a first leg, a second leg and a yoke wherein at least one of the first leg, the second leg and the yoke comprises a plurality of carbon fibers being embedded in a non-conductive matrix.

## Description

### Field of the invention

The present invention relates to an MRI compatible fixation frame, and in particular to an MRI compatible fixation frame having a significantly reduced MR-imaging distorting effect.

### Background of the invention

Medical imaging commonly is used to assist in the diagnosis and/or treatment of patients. Magnetic Resonance Imaging (MRI) is an example of a medical imaging technology that often is used during the diagnosis and treatment of tumors. Treatment of tumors often requires that the radiation treatment of patients is performed at the same time as a magnetic resonance imaging, so that there is a need to use components, which are compatible during both, treatment and imaging of the patient. In particular, it is desired to reduce the impact and distorting effects of the inventory when performing MR-imaging.

In the prior art, several components are known which are designed to be used during MRI. From US 2010/0113913 A1 for example, a magnetic resonance imaging apparatus, a bed device for magnetic resonance imaging apparatus and a tabletop of a bed device for a magnetic resonance imaging apparatus is known, wherein the tabletop comprises plural conductive and elongated material sections placed a distance apart to not contact each other, insulating material sections that are nonmagnetic provided between the plural conductive and elongated material sections to insulate the conductive and elongated sections from each other, and material to hold the conductive and elongated sections and the insulating material sections into a plate sheet shape. The conductive and elongated material sections may be made from carbon fiber reinforced plastics.

Further, from US 2014/0121497 A1, a radio frequency compatible and X-ray translucent carbon fiber and fibrate carbon fiber structure is known, wherein the structure comprises at least two electrically conductive lamina having carbon fibers embedded in a non-conductive matrix, wherein each conductive lamina has an axis perpendicular to the axis of the lamina, and at least one insulating lamina having an axis perpendicular to the plane of the lamina, wherein the conductive lamina are separated by the insulating lamina, wherein the structure is X-ray translucent and does not significantly affect magnetic resonance imaging, X-ray based imaging or other radio frequency dependent applications. A similar technology is also known from US 2013/0078414 A1.

According to US 8,245,335, a support component for use in imaging by magnetic resonance and X-ray is known, wherein the structural support is formed of a fiber reinforced resin material, where the fibers are laid in sheets and infused by the resin material to provide a flat structural member of sufficient strength to carry the weight of a typical patient.

In order to fix a patient during treatment and diagnosis, US 5,730,745 describes a vacuum-assisted fixation apparatus comprising a stereotactic frame and a mouthpiece attached to said stereotactic frame, wherein said mouthpiece having a surface for contacting a patient's hard palate.

US 2014/0059771 A1 describes a table adaptor with joint assembly.

Nevertheless, there remains a need to reduce any relevant impact on the MR-imaging on the one hand and to provide a sufficient fixation and positioning of the patient during radiation treatment of tumors on the other hand. Consequently, there may be a need to provide an MRI compatible frame having a sufficient strength and stiffness to provide a proper positioning on the one hand and to have a minimum impact on the MR-imaging on the other hand.

### Summary of the invention

The present invention provides an MRI compatible fixation frame and components thereof, which reduce the impact on magnetic resonance imaging whilst providing a sufficient fixation of the patient during treatment and imaging.

According to an embodiment, there is provided a magnet resonance imaging (MRI) compatible frame for a mouthpiece-supported patient positioning for use in a high magnetic field environment, wherein the MRI compatible frame comprises a first leg having a first end portion, a second end portion and an intermediate portion longitudinally extending between the first and second end portion of the first leg, a yoke having a first end portion, a second end portion and an intermediate portion longitudinally extending between the first and second end portion yoke, wherein the first end portion of the first leg is connectable to a patient resting structure, wherein the second end portion of the first leg is connected to the first end portion of the yoke structure, wherein at least a first one of the intermediate portion of the first leg and the intermediate portion of the yoke comprises a plurality of carbon fibers being embedded in a non-conductive matrix, wherein the plurality of carbon fibers in the respective intermediate portion are isolated from each other so as to avoid conductive loops.

Thus, an MRI compatible frame can be provided, which has a sufficient strength owing to a carbon fiber reinforced matrix, which is stable and of light weight, and at the same time having a structure, which avoids loops and parts of loops in order to avoid distortions during MR-imaging. Such an MRI compatible frame provides a sufficient structure for stabilizing and positioning of a patient during radiation treatment as well as MR-imaging to avoid radiation impact to not desired areas of the patient and to provide high quality imaging owing to a stabilized patient, and to allow at the same time to have a reduced distortion of the MR-imaging caused by the fixation inventory, here the fixation frame.

According to an embodiment of the invention, the MRI compatible frame further comprises a second leg having a first end portion, a second end portion and an intermediate portion longitudinally extending between the first and second end portions of the second leg, wherein the first end portion of the second leg structure is connectable to a patient resting structure, wherein the second end portion of the second leg structure is connected to the second end portion of the yoke, wherein at least a first one of the intermediate portion of the first leg, the intermediate portion of the second leg and the intermediate portion of the yoke comprises a plurality of carbon fibers being embedded in a non-conductive matrix, wherein the plurality of carbon fibers in the respective intermediate portion are isolated from each other so as to avoid conductive loops.

Thus, an MRI compatible frame can be provided having the properties as mentioned above, whereas the second leg allows to support the yoke on the second end portion. Supporting the yoke with only one leg on one side of the yoke allows to have access to the patient's head from the side of the yoke being opposite to the side where the first leg supports the yoke over the patient resting structure. If such a side access to the patient is not required, the second leg may be provided to support the yoke also on the other side, so that the components of the MRI compatible frame, in particular the first leg, the second leg and the yoke can be made smaller and dimensioned narrower compared to a structure having only the first leg and the yoke.

According to an embodiment of the invention, the plurality of carbon fibers in the respective intermediate portion are substantially beside to each other and do not cross so as to avoid conductive loops.

According to an embodiment of the invention, the plurality of carbon fibers in the respective intermediate portion are substantially parallel to each other so as to avoid conductive loops.

Thus, not only loops, but also parts of loops can be avoided by providing parallel carbon fibers. The respective component does not provide any structure in which the magnetic field of the MR device may induce a current which may distort the imaging process.

According to an embodiment of the invention, the MRI compatible frame further comprises a mouthpiece and a mouthpiece lever, wherein the mouthpiece lever connects the mouthpiece and the yoke, wherein the mouthpiece lever is adaptable to bring the mouthpiece to an appropriate position and orientation with respect to the patient resting on the patient resting structure such that the patient can be brought into a defined position with respect to the MRI compatible frame.

Thus, a patient can be fixed with respect to the resting structure and the frame in that the patient bites onto the mouthpiece. It should be noted that the mouthpiece may be provided with a vacuum volume having an opening which may be covered by the patient's palate, which vacuum volume can be pressure monitored in order to monitor whether the patient still rests in the predetermined fixing position, i.e. the palate still covers to opening which is an indicative for a proper positioning. It should be noted that also the mouthpiece and the mouthpiece lever may be manufactured from a material which does not distort the MR-imaging. Also the mouthpiece and the mouthpiece lever may be reinforced with fibers, for example, carbon fibers, which may be arranged in the mouthpiece and/or mouthpiece lever without contact to each other and without forming a loop as described above, e.g. beside to each other or parallel to each other.

According to an embodiment of the invention, at least a second one of the intermediate portion of the first leg, the intermediate portion of the second leg and the intermediate portion of the yoke comprises a plurality of carbon fibers being embedded in a non-conductive matrix, wherein the plurality of carbon fibers in the respective intermediate section are isolated from each other so as to avoid conductive loops, wherein at least one of the end portions of the first leg, the second leg and/or the yoke being connected to another one of the end portions of the first leg, the second leg and/or the yoke is adapted to avoid an electrically conducting connection between the carbon fibers of the first leg, the second leg and the yoke and the carbon fibers of a connected one of the first leg, second leg and the yoke.

Thus, the respective components, the first leg, the second leg and the yoke, and in particular their end portions are provided with a structure, in particular an isolating structure avoiding any contact of carbon fibers from one to the other component, so that an induced current resulting from the high magnetic field of the MR-imaging is interrupted, so that no or at least a significantly reduced induction takes place.

According to an embodiment of the invention, all of the intermediate portions of the first leg, the intermediate portion of the second leg and the intermediate portion of the yoke comprise a plurality of carbon fibers being embedded in a non-conductive matrix, wherein the plurality of carbon fibers in the respective intermediate section are isolated from each other so as to avoid conductive loops, wherein at least one of the respective end portions of the first leg, the second leg and the yoke being connected to the end portions of the first leg, the second leg and the yoke is adapted to avoid an electrically conducting connection between the carbon fibers of the first leg, the second leg and the yoke and the carbon fibers of a connected one of the first leg, the second leg and the yoke.

According to an embodiment of the invention, the intermediate portion of at least one of the first leg and the second leg follow an offset Z- or S-shape, wherein a section of the intermediate portion close to the first end of the respective leg and a section of the intermediate portion close to the second end of the respective leg are substantially parallel.

According to an embodiment of the invention, the carbon fibers of the intermediate portion of at least one of the first leg, and the second leg follow an offset Z- or S-shape of the respective first leg and second leg.

Thus, the carbon fibers lie beside each other in the respective element, i.e. the first leg and/or the second leg, but do not form loops. Further, a bending into the one direction and the bending into the opposite other direction compensates an inductive effect. At the same time, the first leg and/or the second leg can be provided with an offset section which allows an easier handling of the patient during preparation of the treatment and imaging. Offset with this respect means that the both end portions are not on the same axis, but on parallel axes. It should be noted that alternatively the both end portions may also be aligned to respective axes being inclined to each other.

According to an embodiment of the invention, at least one of the intermediate portions of the first leg, the second leg and the yoke is arc-shaped, wherein the carbon fibers in the respective intermediate portion follow the respective arc shape.

Thus, the frame may be provided not only as a rectangular-shaped frame but also as an arc-shaped frame, but with a minimum induction effect when providing the respective elements of the frame with carbon fibers to reinforce the structure to provide a stronger and reinforced structure. Although the entire frame may be arc-shaped, the respective portions of a first leg, a second leg and the yoke have only a very limited bending. As the carbon fibers of the first leg, the second leg and the yoke are isolated from each other, no current occurs from, for example, the first leg to the yoke, owing to the isolated end portions, so that the entire inductive effect of the arc-shaped frame remains low. It should be noted that the fibers in the arc-shaped components may also be arranged in parallel instead of following the bending.

According to an embodiment of the invention, each of the intermediate portions of the first leg, the second leg and the yoke are arc-shaped, wherein the first leg, the second leg and the yoke together form a substantially continuously bended arc.

Thus, although the frame has a form of an arc which may be considered as a half winding of a coil, the isolation between the respective sections of the arc avoid a current flow and thus an inductive effect during MR-imaging.

According to an embodiment of the invention, at least one of a first leg, the second leg and the yoke comprises at least two separated and isolated longitudinal subsections, being aligned in a longitudinal direction of the respective intermediate portion, wherein each of the longitudinal subsections comprises a plurality of carbon fibers being embedded in a non-conductive matrix, wherein the plurality of carbon fibers in the respective longitudinal subsection are isolated from each other so as to avoid conductive loops, wherein the subsections of a respective first leg, second leg and/or yoke are electrically isolated with respect to each other.
Thus, it is possible to design the first leg, the second leg and/or the yoke having a more complex structure which may deviate from a pure straight shape, but to maintain the isolated parallel carbon fibers in respective subsections and the isolation between the carbon fibers between adjacent subsections. Thus, it is also possible to provide, for example, a loop-shaped yoke, wherein the subsections in such a yoke are separated from each other so as to avoid a closed conducting loop in the inside of the yoke. Further, it is possible to provide strictly straight parallel carbon fibers, even if the first leg, the second leg and/or the yoke are bended.

According to an embodiment of the invention, at least one of the first leg, the second leg and the yoke comprises at least two separated and isolated longitudinally extending subsections being laterally aligned with respect to a longitudinal direction of the respective intermediate portion, wherein each of the lateral subsections comprises a plurality of carbon fibers being embedded in a non-conductive matrix, wherein the plurality of carbon fibers in the respective subsection are isolated from each other so as to avoid conductive loops, wherein the subsections of a respective one of the first leg, second leg and/or yoke are electrically isolated with respect to each other.

Thus, not only complex structures with longitudinally aligned subsections but also laterally arranged subsections are possible to provide more complex structures of frame components.

According to an embodiment of the invention, at least one of the end portions of the first leg, the second leg and the yoke comprise a coupling member matching a cooperating coupling member of another one of the end portions of the first leg, the second leg and the yoke coupled thereto, wherein the matching coupling members can be brought from a locked state into a releasable state and vice versa.

Thus, it is possible to provide different types of legs and yokes in order to combine those legs and yokes to get a customized frame being composed of legs and yokes of different dimensions. The locked state provides a fixed mounting of the elements in order to provide a stable and rigid frame, wherein the released state allows a disassembling and reassembling according to the requirements of the patient. Further, such a component system allows to easily build up the frame in case the patient is already resting on the patient rest. It should be noted that the coupling members may also be provided to couple the end portions of the respective leg to the resting structure. If it is desired to maintain a predetermined orientation of the components, the coupling members may have a unique coupling geometry allowing assembly only in the predetermined combination. In case the orientation is without relevance, e.g. the respective component is symmetrical, the coupling geometry on both ends may be identical, which may simplify the assembly of the frame.

According to an embodiment of the invention. The coupling member comprises an arrangement of pins and/or holes matching an arrangement of pins and/or holes of a cooperating coupling member, which is intended to be connected to the coupling member.

Thus, it is possible to provide a support for assembling the frame and to further stabilize the coupling area of the frame to provide a rigid frame structure. The mentioned pins and/or holes may be used to provide either an identical connecting structure and geometry or a unique coupling structure or geometry for reasons as mentioned above.

According to an embodiment of the invention, at least one of the end portions of the first leg, the second leg and the yoke comprises a fast lock connector being adapted to bring the matching coupling members from a released state into a locked state by applying a pressing force in a direction which corresponds to an insertion direction of one of the pins into one of the holes.

Thus, the assembly can be accelerated, which may reduce the preparation time. The fast lock connector may also be designed as a snap connector which automatically transits into a fixed lock state upon pressing two elements to each other, so that no additional actions may be required to lock the coupling portions. This accelerated assembly and requires "less hands".

According to an embodiment of the invention, the first leg, the second leg and the yoke are adhesively connected to each other, wherein an adhesive agent forms an electrical isolation between the first leg and the yoke as well as the second leg and the yoke.

Thus, in case the frame is provided as an integral frame without the need for disassembling, the respective portions can be isolated from each other whilst providing a proper and stable connection between the elements of the frame, i.e. the first leg, the second leg and the yoke. It should be noted that the first leg and the second leg may have releasable connectors for mounting the frame onto the resting structure.

According to an embodiment of the invention, each of the first leg, the second leg and/or the yoke is provided with an arrangement of markers, such that the spatial orientation of the MRI compatible frame is uniquely recognizable in a fluoroscopic projection or MR-imaging.

According to an embodiment of the invention, the entirety of the first leg, the second leg and the yoke is provided with an arrangement of markers, such that the spatial orientation of the MRI compatible frame is uniquely recognizable in a fluoroscopic projection or MR-imaging.

Thus, it is possible to determine the position of the first leg, the second leg and/or the yoke or the entire frame with respect to the monitored and imaged patient and to use the frame or its components as a reference for determining the position of patient's structures with respect to the frame geometry. It should be noted that also the first leg, the second leg and the yoke as separate components may be provided with an arrangement of markers, so that each of the first leg, the second leg and the yoke are separately uniquely recognizable in a fluoroscopic projection or MR-imaging, and each alone may be used as a reference body. Further, it should be noted that if the first leg, the second leg and/or the yoke are designed as symmetrical components, which means that the assembling orientation is without relevance, also a respective symmetrical marker arrangement can be used, so that the marker structure remains the same regardless of the mounting orientation of the first leg, the second leg and/or the yoke.

According to an embodiment of the invention, the marker arrangement is a fiducial marker arrangement having a unique 2-dimensional projection in at least a predetermined spatial range, preferably in all spatial directions.

According to an embodiment of the invention, the marker arrangement is an infrared marker arrangement having a unique 2-dimensional projection in at least a predetermined spatial range, preferably in all spatial directions.

According to an embodiment of the invention, the marker arrangement comprises a combination of fiducial markers and IR-markers, wherein preferably at least a part of the markers are designed to be a fiducial marker and an IR-marker at the same time.

According to an embodiment of the invention, the first leg, the second leg and the yoke at least partially are manufactured from an insulating material, which has a very small distortion of a homogeneous B0 magnetic field of less than 0.25 ppm inside a relevant MR-imaging volume so as to not generate unacceptable distortion in MR-images.

Thus, even if particular components of the first leg, the second leg and the yoke are not manufactured with a carbon fiber reinforcement with carbon fibers being embedded in a non-conductive matrix, those remaining structures may be made of a pure resin or plastic or ceramic or wood material, which remains inactive in a magnetic field. It should be noted that for a certain rigidity or stable structure of a frame, it may be sufficient to provide the first leg and the second leg with a carbon fiber reinforced structure being embedded in a non-conductive matrix, whereas the yoke may be made of a material which is not fiber reinforced.

It should be noted that although the prior art already describes resting structures being made of carbon fiber reinforced material, in particular carbon fibers being embedded in a non-conductive matrix, the present invention provides a carbon fiber structure being embedded in a non-conductive matrix for a fragile frame structure which needs to be rigid and stable to avoid patient movement and to guarantee fixation of the patient's head on the one hand, and to allow an easy handling of the patient and accessibility to the patient by providing a slim structure of the respective elements, i.e. the first and second leg and the yoke. The carbon fibers may be avoided when providing a broader leg or yoke, however this would significantly reduce the accessibility to the patient resting on the resting structure and being surrounded by the frame.

In other words, the invention provides a carbon block structure being comprised of many carbon fiber elements in a non-conductive block. In this way, all conductive carbon elements in each carbon block are parallel with each other and cannot form any current loop under MR environment. Since each carbon fiber block is electrically isolated over another carbon fiber block, the overall frame cannot form a current loop under MR environment. The insulated material or non-conductive matrix may be plastic, epoxy resin and/or polyester, polymer, polymethacryl amide, vinyl ester resin, wood, ceramic, aramid, glass fiber, ultra high molecular weight polyethylene, etc. As a consequence, the frame still benefits the original carbon fiber reinforcement character, e.g. to be rigid, having a low density and less attenuation with X-ray or other radiotherapy. Even in case the carbon elements are not straight, but still beside each other, which means do not contact each other, a current loop may be avoided and therefore will not cause MRI artefacts.

It should be noted that embodiments as described above may be combined with respect to each other so as to gain a synergetic effect, which may extend over the separate technical effects of the single features. Exemplary embodiments of the present invention will be described in the following.

### Brief description of the figures

Exemplary embodiments of the invention will be described in the following with reference to the following figures.
- Figure 1: illustrates an MRI compatible fixation frame according to an exemplary embodiment of the invention.
- Figure 2: illustrates a schematic inside view of a frame section/component according to an exemplary embodiment of the invention.
- Figure 3: illustrates an MRI compatible fixation frame having a mouthpiece mounted thereon according to an exemplary embodiment of the invention.
- Figure 4: illustrates a frame element, here a first/second leg following an S-shape according to an exemplary embodiment of the invention.
- Figure 5: illustrates an element of an MRI compatible fixation frame, here a first/second leg following a Z-shape according to an exemplary embodiment of the invention.
- Figure 6: illustrates a schematic inside view of a joint between two elements of an MRI compatible fixation frame according to an exemplary embodiment of the invention.
- Figure 7: illustrates an arc-shaped MRI compatible fixation frame according to an exemplary embodiment of the invention.
- Figure 8: illustrates a schematic inside view of a first/second leg having longitudinal and lateral subsections according to an exemplary embodiment of the invention.
- Figure 9: illustrates a schematic inside view of a first/second leg having separated subsections according to an exemplary embodiment of the invention.
- Figure 10: illustrates an inside view of a yoke having lateral and longitudinal aligned subsections according to an exemplary embodiment of the invention.
- Figure 11: illustrates a schematic inside view of a bended yoke having longitudinally aligned subsections according to an exemplary embodiment of the invention.
- Figure 12: illustrates an MRI compatible fixation frame having a marker arrangement thereon according to an exemplary embodiment of the invention.
- Figure 13: illustrates an MRI compatible fixation frame with a mouthpiece mounted thereon and having a marker arrangement thereon according to an exemplary embodiment of the invention.
- Figure 14: illustrates a joint/connection between a first/second leg and yoke according to an exemplary embodiment of the invention.
- Figure 15: illustrates a further joint/connection between a first/second leg and yoke according to an exemplary embodiment of the invention.
- Figure 16: illustrates a quick lock joint/connection between a first/second leg and a yoke according to an exemplary embodiment of the invention.

### Detailed description of exemplary embodiments

In the following, a detailed description of exemplary embodiments will be given to explain the invention in more detail.

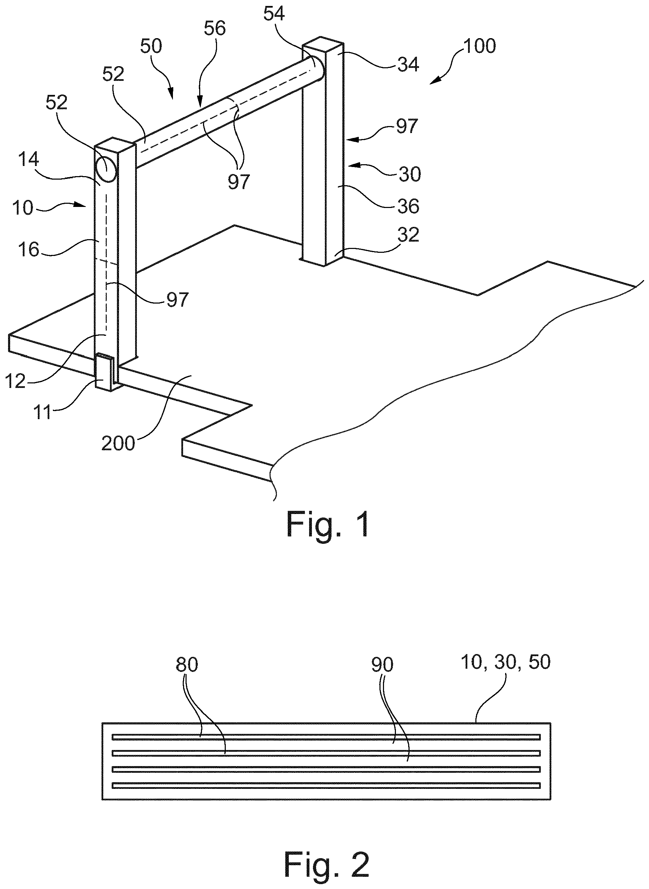

Figure 1 illustrates a magnet resonance imaging MRI compatible frame according to an exemplary embodiment of the invention. As can be seen from Figure 1, the MRI compatible fixation frame 100 can be fixed to a patient resting structure 200 where a patient rests during radiotherapy and MR imaging during radiotherapy treatment. A magnetic resonance imaging compatible frame may be used to mount components to support a fixation and/or positioning of a patient with respect to the frame 100 or the patient resting structure 200. In order not to overburden Figure 1 with elements to be mounted to the frame 100, such components are not shown in Figure 1. The MRI compatible fixation frame 100 comprises a first leg 10, a second leg 30 and a yoke 50. The first leg 10 comprises a first end 12 and a second end portion 14, wherein the first leg 10 is mounted to the patient resting structure 200 with the first end portion 12. It should be noted that the mounting to the patient resting structure 200 may be releasable and may be secured for example with a coupling member 11. In the same manner, the second leg 30 comprises a first end portion 32 and a second end portion 34. The second leg 30 may be mounted to the patient resting structure 200 with the first end portion 32. The yoke 50 has a first end portion 52 and a second end portion 54, wherein the first end portion 52 of the yoke 50 is connectable to the second end portion 14 of the first leg 10. Correspondingly, a second end portion 54 of the yoke 50 is connectable to the second end portion 34 of the second leg 30. In the embodiment illustrated in Figure 1, the first leg 10 and the second leg 30 have a square or rectangular cross-section and the yoke 50 has a circular or elliptic cross-section. However, the invention is not limited to the illustrated cross-sections. As will be described in the following, the yoke 50 may also have a square or rectangular cross-section. Each of the first leg 10, the second leg 30 and the yoke 50 have an intermediate portion 16, 36, 56. The intermediate portion 16 of the first leg 10 extends between the first end portion 12 and the second end portion 14 of the first leg. The intermediate portion 36 of the second leg 30 extends between the first end portion 32 and the second end portion 34 of the second leg 30. The intermediate portion 56 of the yoke 50 extends between the first end portion 52 and the second end portion 54 of the yoke 50. Although not illustrated in detail, the joint between the second end portion 14 of the first leg 10 and the first end portion 52 of the yoke 50 and the corresponding joint between the second end portion 34 of the second leg 30 and the second end portion 54 of the yoke 50 may be designed as a releasable junction or may be designed as a fixed junction. The inventory to be used for fixation and positioning of a patient may be mounted to the yoke 50. However, also the legs 10, 30 may be used to fix inventory. The MRI compatible frame 100 may have markers 97 on the frame. The markers 97 may be used for a laser scanner or camera to recognize the position and orientation of the frame. For this purpose, the optical markers 97 may be provided on an outward directing face of the MRI compatible frame 100, as can be seen in Figure 1. Thus, it is possible that a laser scanner or camera can recognize the respective frame portions by detecting the laser markers 97, which are cross shaped according to the embodiment illustrated in Figure 1. However, it should be noted that such markers may have different forms although not illustrated in detail.

It should be noted, that the frame illustrated in Figure 1 also may have only one single leg and the yoke 50. Consequently, e.g. the second leg 30 may be left out. As far as the first leg 10 and the yoke 50 are dimensioned so as to provide a sufficient stability and stiffness, the second leg 30 may be left out, so that one side of the frame remains open. This allows it to have an easier access to a patient's head resting beneath the yoke 50. In particular, it is also possible to move the patient beneath the yoke 50 from the lateral side.

The elements of the MRI compatible frame 100, in particular the first leg 10, the second leg 30 and the yoke 50 may have a particular structure, as will be described in detail in the following. Figure 2 illustrates a schematic inside view or sectional view of the components of the MRI compatible frame, here exemplarily the first leg 10 or the second leg 30 or the yoke 50. A plurality of carbon fibers 80 may be provided within the respective element 10, 30, 50, wherein the respective carbon fiber elements are embedded in a non-conductive matrix 90, so that the carbon fibers 80 are isolated from each other. Thus, it can be avoided that carbon fibers 80 form loops by accidentally contacting each other which then will generate distortions during MR Imaging. It should be noted, that carbon fibers 80 may also be bundles or woven threads of carbon fibers, which threads may have a weaver pattern which provides compensating loops so as to directly compensate any inductive impact on a carbon fiber thread. As will be described later, the carbon fibers 80 may be provided in sections or subsections.

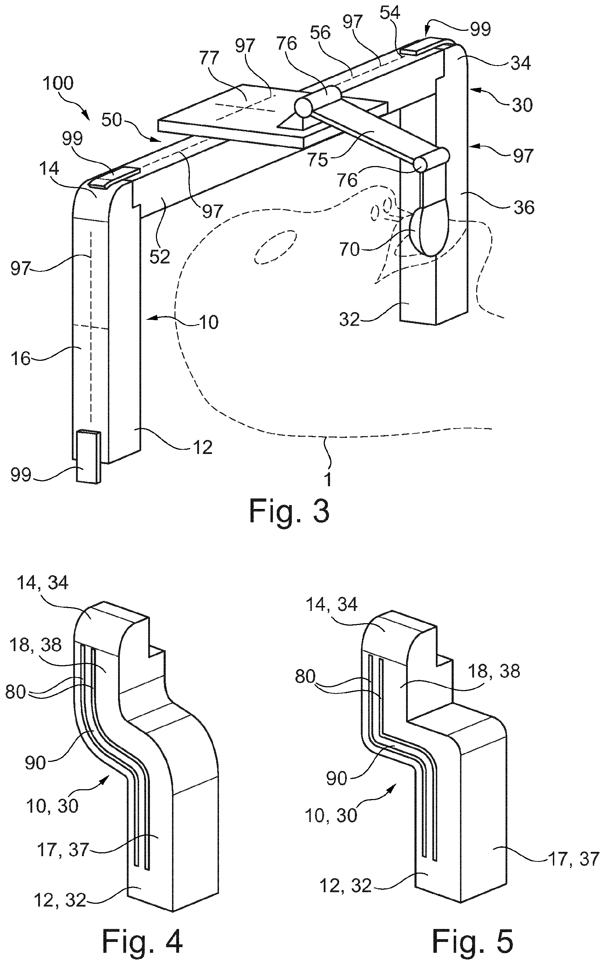

Figure 3 illustrates a further exemplary embodiment of an MR compatible frame 100. The frame 100 illustrated in Figure 1 comprises the first leg 10, the second leg 30 and the yoke 50. The first leg may be coupled to the yoke 50 with a fast lock connector 99, which allows it to easily lock and release the first leg 10 from the yoke 50. Correspondingly, the second leg 30 may be connected to the yoke 50 by a fast lock connector 99. Although not illustrated in detail, the first and second leg 10, 30, may have a fast lock connector 99 on the respective first end portion 12, 32 in order to connect the first leg 10 and the second leg 30 to a not illustrated patient resting structure 200. The frame 100 may have a mouthpiece 70 mounted thereon, wherein the mouthpiece according to the embodiment illustrated in Figure 3 is connected to a mounting plate for the mouthpiece 77, wherein the mouthpiece 70 is connected to the mouthpiece mounting plate 77 by a mouthpiece lever 75. The mouthpiece 70 may be connected to the mouthpiece lever 75 by a hinged connection 76. Correspondingly, the mouthpiece lever 75 may be connected to the mouthpiece mounting plate 77 by a hinged connection 76. Thus, it is possible to position the mouthpiece 70 with respect to a patient 1 resting on the patient resting structure (not illustrated). It should be noted that the mouthpiece lever 76 may have a further hinged connection (not illustrated) in order to increase the flexibility of positioning of the mouthpiece 70 with respect to the patient. The hinged connections 76 may be fixed after positioning, so that the entire structure including the mouth piece 70, the lever 75and the mounting plat 77 may be made rigid for maintaining the position of a patient. A patient may take the mouthpiece 70 into the mouth, whereas the mouthpiece 70 may be individually adapted to the individual mouth anatomy of the patient in order to provide a sufficient matching for reliably positioning of the patient 1. As explained with respect to Figure 1, the frame illustrated in Figure 3 may include laser/optical markers 97 on the legs 10, 30 as well as the yoke 50 and the mouthpiece (not illustrated) or the mouthpiece plate 77. At least one of the first leg 10, the second leg 30 and the yoke 50 may have an internal structure which was described with respect to Figure 2. By providing parallel and isolated carbon fibers 80 in a non-conductive/isolating matrix 90, loops of carbon fibers may be avoided, resulting in a low distortion of the MR Imaging. With this respect, the frame can be made rigid by using carbon fibers whilst maintaining the frame slim, as carbon fibers and carbon fiber reinforced materials have a sufficient strength at moderate cross-sections. As carbon fibers are conductive, loops of carbon fibers may occur when putting carbon fibers as bundles into a resin. The magnetic field applied during radiotherapy or MR imaging may induce currents in such loops, which currents then will generate a magnetic field. Such a generated magnetic field may seriously influence the quality of MR imaging, so that avoiding loops in the carbon fiber structure avoids inductive currents, so that a distorting magnetic field generation can be suppressed. It should be noted that this structure of carbon fibers 80 embedded into a non-conductive matrix 90 may be provided in one or two legs in order to provide a sufficient support for a yoke 50. If it is required for stability reasons, also the yoke 50 may be provided with such a carbon fiber structure 80 embedded into a non-conductive matrix 90. However, it should be noted, that if both legs 10 and 30 provide a sufficient stability, the yoke 50 may also be manufactured of a non-conductive material without carbon fibers 80 embedded therein. Of course, all elements of an MRI compatible frame 100 may be provided with such a carbon fiber 80 embedded into a non-conductive matrix 90 structure as illustrated. With this respect, it should be noted that the structure of Figure 2 is only a schematic structure and that the actual dimensions of the carbon fibers 80 and the matrix structure 90 may differ from that in Figure 2. It should be noted, that carbon fibers may be fiber-by-fiber embedded into the matrix structure 90, for example by applying a liquid resin which hardens later on. However, it is also possible to coat the carbon fibers 80 in advance, then to bundle the coated and thereby isolated carbon fibers 80 and to embed a bundle of such carbon fibers 80 into the isolating or non-conductive matrix structure 90.

Figure 4 illustrates an exemplary embodiment of a first or second leg 10, 30 wherein the leg illustrated in Figure 4 has a non-straight shape. According to the embodiment illustrated in Figure 4, the leg has an S-shape, whereas a first end sided section 17, 37 of the intermediate portion 16, 36 is oriented toward the first end portion 12, 32 of the leg 10, 30, whereas a second sided section 18, 38 of the intermediate portion 116, 36 is oriented toward the second end portion 14, 34 of the leg 10, 30. The carbon fibers 80 embedded into the non-conductive matrix 90 may follow the S-shape of the leg 10, 30. The carbon fibers 80 in the first end sided section of the intermediate portion 17, 37 are almost parallel to the carbon fibers 80 in the second end sided section of the intermediate portion 18, 38. It should be noted, that the carbon fibers 80 remain isolated from each other by the non-conductive matrix 90.

Figure 5 illustrates a further exemplary embodiment of the first/second leg 10, 30, wherein the leg follows a Z-shape. The Z-shape differs from the S-shape in that the Z-shape has a sharp bend, whereas the S-shape has a smooth bend. Consequently, the carbon fibers 80 may follow the Z-shape of the respective leg 10, 30, whereas the first end sided section 17, 37 of the intermediate portions are parallel to the second end sided sections 18, 38 of the intermediate portions. Accordingly, the respective portions of the carbon fibers 80 are parallel. It should be noted, that the carbon fibers 80 remain separated and isolated from each other by the non-conductive/isolating matrix 90.

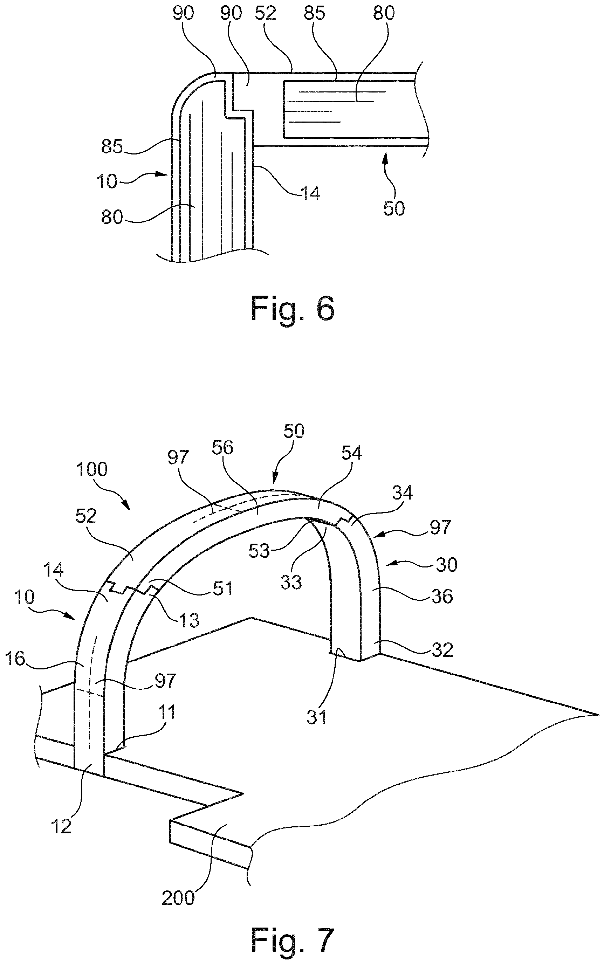

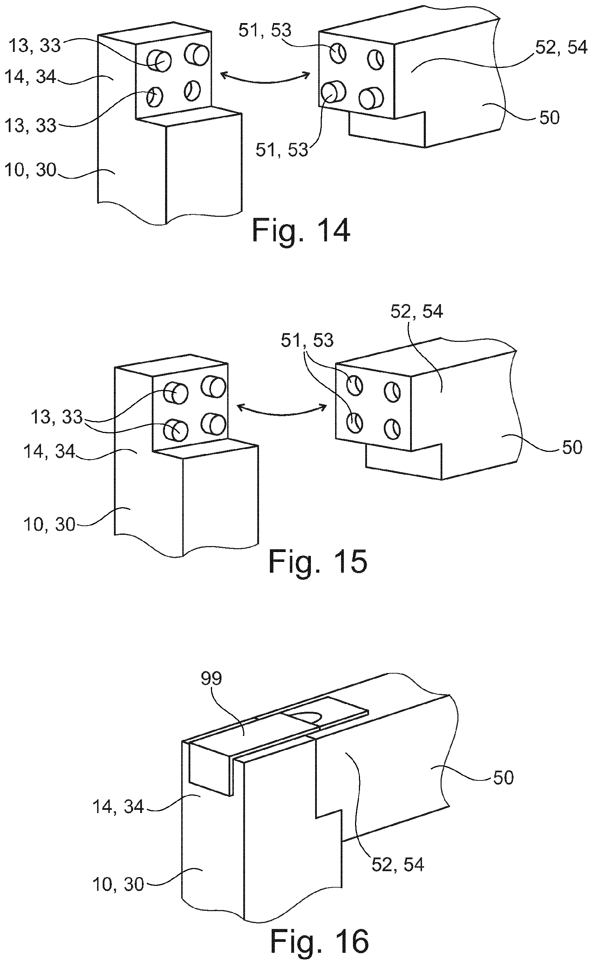

Figure 6 illustrates a schematic cross-sectional view of a cutout of a frame, wherein the first leg 10 is connected to the yoke 50. The carbon fibers 80 may be provided as a carbon fiber bundle or a carbon fiber block 85, which may be embedded into a non-conductive matrix 90. It should be noted, that a carbon fiber bundle or carbon fiber block 85 may be manufactured by bundling pre-coated carbon fibers 80 in order to establish isolation of the carbon fibers or by embedding carbon fibers into a non-conductive matrix forming the matrix for the carbon fiber bundle or the carbon fiber block 85. As can be seen in Figure 6, a respective first end portion 52 of the yoke 50 may have a particular shape to match the corresponding second end portion 14 of the first leg 10 in order to provide a sufficient stable connection between the first leg 10 and the yoke 50. It should be noted, that the shape of the end portions of the legs and the yoke may have a different shape according to the requirements, as will be described in detail with respect to Figures 14 and 15 later on.

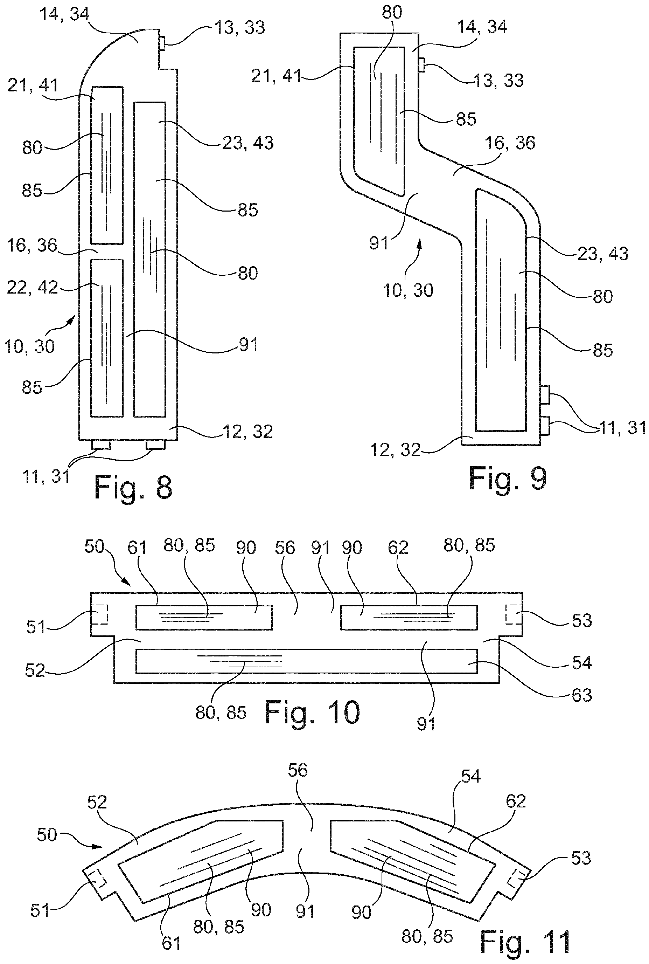

Figure 7 illustrates a further exemplary embodiment of an MRI compatible frame 100. The frame 100 illustrated in Figure 7 has a generally arc-shaped form. Although the arc follows an almost continuous bending along the frame, the frame may be separated in a first and second leg 10, 30 and a yoke 50. In this embodiment illustrated in Figure 7, the legs 10, 30 as well as the yoke may be bent in order to form an almost continuously bending shape of the arc. As can be seen in Figure 7, the first end portion 52 of the yoke 50 may match a corresponding shape of the second end portion 14 of the first leg 10. The same applies to the second end portion 54 of the yoke 50 and the second end portion 34 of the second leg 30. The respective end portions 14, 12, 34, 32 may be provided with coupling members 13, 11, 33, 31 in order to provide a sufficient coupling of the legs to the yoke 50 as well as the legs 10, 30 to patient resting structure 200. As described with respect to Figure 1, also the arc-shaped frame of Figure 7 may be provided with laser/optical markers 97, which may be provided on the legs as well as on the yoke. At least one of the first leg 10, second leg 30 and the yoke 50 may have an internal structure having carbon fibers 80 embedded into a non-conductive matrix 90. The carbon fibers 80, although isolated over each other, may follow the arc-shaped bending of the legs 10, 30 as well as the yoke 50. However, although the outer shape of the legs 10, 30 and the yoke 50 are arc-shaped, the internal structure may provide carbon fibers 80 which are arranged in a straight direction, as will be described in detail with respect to Figure 11 later on. It should be noted, that the illustration of Figure 11 applies to the legs 10, 30 as well as the yoke 50. The structure of the carbon fibers 80 embedded into a non-conductive/isolating matrix 90 may be provided in the intermediate portions of the first leg, the second leg and the yoke 16, 36, 56. Although not illustrated in detail, the mounting of the end portions of the legs as well as the end portions of the yoke may be achieved by a fast lock connector, which is not illustrated in detail in Figure 7. The same applies to the end portions of the legs with respect to the mounting to the patient resting structure 200. It should be noted that the frame illustrated in Figure 7 may also be divided into four or more components. When dividing the arc into four sections, the both middle sections may be considered as a yoke being composed of two sections. It should also be noted that the arc may be divided into only two sections, which may be interpreted as having only the legs, but no yoke.

Figure 8 illustrates the further exemplary embodiment of a leg 10, 30. The leg illustrated in Figure 8 has a straight shape, whereas also the carbon fibers 80 run in a straight direction. The leg 10, 30 may be provided with longitudinally arranged carbon fiber sections and/or laterally aligned carbon fiber sections. In Figure 8, the leg 10, 30 may have a first carbon fiber section 21, 41 and a second carbon fiber section 22, 42 which are aligned in a longitudinal direction. Additionally, or alternatively, leg 10, 30 may have a third carbon fiber section 23, 43 which is aligned laterally to the first and/or second carbon fiber sections 21, 41, 22, 42. Each of the carbon fiber sections may be built up as a carbon fiber bundle 85 or a carbon fiber block which may be pre-manufactured by embedding carbon fibers 80 into a non-conductive or isolating matrix 90. It should be noted, that the carbon fiber blocks 85 may also be embedded into a non-conductive/isolating matrix 90, which may constitute a non-conductive isolating intermediate section 91 between the carbon fiber sections described above. Figure 8 further illustrates, regardless of the internal structure of the carbon fibers and carbon fiber sections, coupling members 11, 31 on the first end portion 12, 32 of the respective leg 10, 30 and a coupling member 13, 33 on the second end portion 14, 34 of the respective leg 10, 30. These coupling portions may be used to couple the respective end portion to either a yoke or to a patient resting structure, which are not illustrated in Figure 8. In Figure 8, the coupling members/structures are illustrated as pins protruding from the end portions.

Figure 9 illustrates a further exemplary embodiment of a leg 10, 30, which has a first carbon fiber section 21, 41 and a third carbon fiber section 23, 43. Both of the carbon fiber sections may have a structure with carbon fibers 80 embedded into a non-conductive or isolating matrix 90 and may be manufactured as preformed carbon fiber bundles/carbon fiber blocks 85 as described above. The intermediate portion 16, 36 may further include a non-conductive/isolating intermediate section 91 to separate the first carbon fiber section 21,41, from the third section 23, 43. A second section 22, 42 is not illustrated in Figure 9. The leg in Figure 9 has a Z-shape, however, the above description also applies to the S-shape.

Figure 9, regardless of the shape and regardless of the internal structure provides coupling members 11, 31 on the first end portion 12, 32 and coupling members 13, 33 at the second end portion 14, 34. Compared to Figure 8, where the coupling members 11, 31 on the second end 14, 34 extend into a downward direction in Figure 9 the coupling members 11, 31 extend into a lateral direction. Although in Figure 8 and in Figure 9 protruding coupling members in the form of a pin are illustrated, also coupling members in the form of a hole or a recess may be provided as will be described later on with respect to Figure 10.

Figure 10 illustrates an exemplary embodiment of a yoke 50 having embedded therein a first carbon fiber section 31, a second carbon fiber section 32 and a third carbon fiber section 33. It should be noted, that a yoke 50 may have embedded into the intermediate portion 56 longitudinally aligned carbon fiber sections like carbon fiber sections 61 and 62 and/or laterally aligned carbon fiber sections like carbon fiber sections 63 and 62 or 63 and 61. The yoke 50 illustrated in Figure 10 has coupling portions 51, 53 at the respective end portions 52, 54. As can be seen in Figure 10, the coupling portions 51, 53 are designed as recesses or bore holes. The holes 51, 53 may correspond to the protruding pins 13, 33 of Figure 8 or Figure 9. Thus, a yoke 50 according to Figure 10 may be coupled to a leg 13, 30 according to Figure 8 or Figure 9 so as to form a frame according to an exemplary embodiment of the invention. As can be seen, the carbon fibers 80 of the yoke 50 and the carbon fibers of the leg 10, 30 will not be in contact with each other, so that the carbon fibers of yoke 50 are isolated over the carbon fibers of leg 10, 30. The same applies to the yoke section of Figure 11 which will be described in the following. It should be noted that the coupling portions may have any other shape and are not limited to circular cross section pins and holes.

Figure 11 illustrates a bended yoke 50. With respect to the coupling, the same applies to the embodiment of Figure 11, and the embodiment of Figure 10. It appears reasonable, to couple the bended arc-shaped yoke 50 of Figure 11 to corresponding arc-shaped legs, which are not illustrated in detail. As can be seen in Figure 11, the bended yoke may have embedded into the intermediate portion 56 two (or more) carbon fiber bundles/carbon fiber blocks 85, where the carbon fibers 80 run straight, although the yoke 50 has a bended shape. Thus, although the yoke 50 is arc-shaped, any bended carbon fibers 80 may be avoided in order to minimize artefacts in MR imaging. With respect to the buildup of the carbon fiber bundles/carbon fiber blocks 85, the description of Figure 10, Figure 8, Figure 9 and Figure 2 applies correspondingly.

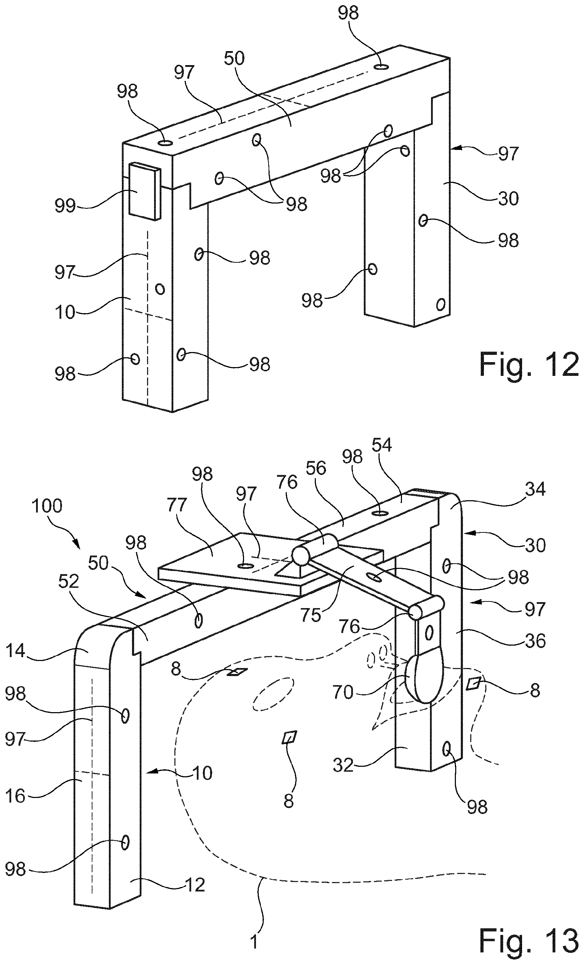

Figure 12 illustrates an MRI compatible frame 100 as described above, having implemented markers. The markers illustrated in Figure 12 are laser/optical markers 97 which have been described with respect to Figure 1. Further, as an alternative or additionally, fiducial markers or infrared markers 98 may be provided onto or within the frame. The fiducial or infrared marker arrangement may be a single marker but may also be a plurality of markers. A plurality of markers may be arranged such that they have a unique two-dimensional projection in at least a pre-determined spatial range of directions, preferably in all spatial directions. The two-dimensional projection may be achieved by an X-ray imaging or MR imaging. This allows provision of the determination of the orientation and spatial positioning of the frame, although only a single two-dimensional image needs to be provided. It should be noted, that the markers may be fiducial markers which can be seen in an X-ray imaging or an MR-imaging as well as infrared markers, which can be seen in an infrared imaging. The fiducial markers may result in a two-dimensional projection image, whereas the infrared markers owing to the reflection may result in a two-dimensional reflection image which may allow determination of the spatial position and orientation of the frame from a single two-dimensional image. For this purpose, the infrared markers may have a reflecting characteristic in that they reflect infrared light back to the direction of the infrared light source. It should be noted, that the at least a part of the markers may be combined markers being combined from fiducial markers and infrared markers. This means, that at least a part of the markers may be designed to be a fiducial marker and an infrared marker at the same time.

Figure 13 illustrates a further exemplary embodiment of MRI compatible frame being provided with a mouthpiece and a mouthpiece mounting, which had been described with respect to Figure 3 in detail. Figure 13 illustrates that the frame may be provided with markers 98 which may be fiducial markers and/or infrared markers as described with respect to Figure 12. Also the mouthpiece mounting plate 77, the mouthpiece lever 75 as well as the mouthpiece as such may have one or more infrared or fiducial marker thereon. In addition to the markers provided on the frame or the elements mounted to the frame, further markers 8 may be provided on or inside of the patient, for example infrared markers or fiducial markers. Such markers may be attached to the forehead, the nose, the cheeks or the chin or within the mouth of the patient. Thus, it is possible to detect the spatial position of the patient with respect to the spatial position of the frame in order to monitor the position and fixation of the patient with respect to the frame.

Figure 14 illustrates a coupling structure between the leg 10, 30 and the yoke 50 according to an embodiment of the invention. As can be seen from Figure 14, the coupling member 13, 33 may have one or more pins and/or holes in order to match corresponding pins and holes of a coupling member 51, 53 of the first or second end portion 52, 54 of the yoke 50. The pins and holes may have an arrangement so that the uniquely fit to each other, which allows provision of a pin/hole coding which only matches on corresponding end portions which are intended to be coupled. This reduces the risk to assemble the frame wrongly. Figure 14 illustrates an embodiment, wherein at the leg, the upper coupling members are pins and the lower coupling members are holes, whereas at the yoke site, the upper coupling members are holes and the lower coupling members are pins so that these pins/hole arrangements directly match the corresponding pin/hole arrangement of the leg.

Figure 15 illustrates an alternative coupling arrangement, wherein on the leg side 10, 30, only pins are provided as coupling members 13, 33 whereas on the yoke side 50 only holes are provided 51, 53 so that they uniquely match to the corresponding leg. As can be seen, the yoke 50 of Figure 14 does not match the leg of Figure 15, so that an unintended connection can be avoided.

If it is intended to couple the legs and the yoke permanently, the legs and the yoke can be connected to each other by a non-conductive glue.

Figure 16 illustrates a fast-lock connector 99 which is used to connect end portions of the leg with the end portions of the yoke. Figure 16 exemplarily illustrates the connection of the second end portion 14, 34 of the leg 10, 30 with the first or second end portion 52, 54 of the yoke 50. The fast-lock connector 99 may be used to quickly snap the connection and to secure the connection between the yoke and the leg. The fast-lock connector may be designed to automatically snap and secure both components to be connected upon positioning of both components in the correct position with respect to each other.

### Used references:

- 1: patient
- 8: marker on or inside of patient, e.g. IR-marker or fiducial marker
- 10: first leg
- 11: coupling member of first end portion of first leg, e.g. pin and/or hole
- 12: first end portion of first leg
- 13: coupling member of second end portion of first leg, e.g. pin and/or hole
- 14: second end portion of first leg
- 16: intermediate portion of first leg
- 17: first end sided section of intermediate portion of first leg
- 18: second end sided section of intermediate portion of first leg
- 21: first carbon fiber section of first leg
- 22: second carbon fiber section of first leg longitudinally arranged to first
- 23: third carbon fiber section of first leg laterally arranged to first
- 30: second leg
- 31: coupling member of first end portion of second leg, e.g. pin and/or hole
- 32: first end portion of second leg
- 33: coupling member of second end portion of second leg, e.g. pin and/or hole
- 34: second end portion of second leg
- 36: intermediate portion of second leg
- 37: first end sided section of intermediate portion of second leg
- 38: second end sided section of intermediate portion of second leg
- 41: first carbon fiber section of second leg
- 42: second carbon fiber section of first second leg longitudinally arranged to first
- 43: third carbon fiber section of second leg laterally arranged to first
- 50: yoke
- 51: coupling member of first end portion of yoke, e.g. pin and/or hole
- 52: first end portion of yoke
- 53: coupling member of second end portion of yoke, e.g. pin and/or hole
- 54: second end portion of yoke
- 56: intermediate portion of yoke
- 61: first carbon fiber section of yoke
- 62: second carbon fiber section of yoke longitudinally arranged to first
- 63: third carbon fiber section of yoke laterally arranged to first
- 70: mouthpiece
- 75: mouthpiece lever
- 76: hinges of mouthpiece lever
- 77: mounting plate of mouthpiece/mouthpiece lever
- 80: carbon fiber(s)
- 85: carbon fiber bundle / carbon fiber block
- 90: non-conductive/isolating matrix/resin
- 91: non-conductive/isolating intermediate section
- 97: laser/optical marker on frame
- 98: marker on frame, e.g. IR-marker or fiducial marker
- 99: fast lock connector
- 100: MRI compatible fixation frame
- 200: patient resting structure

## Claims

1. Magnet resonance imaging (MRI) compatible frame for mouthpiece-supported patient positioning for use in a high magnetic field environment, the MRI compatible frame (100) comprises:
a first leg (10) having a first end portion (12), a second end portion (14) and an intermediate portion (16) longitudinally extending between the first and second end portion of the first leg;
a yoke (50) having a first end portion (52), a second end portion (54) and an intermediate portion (56) longitudinally extending between the first and second end portion of the yoke;
wherein the first end portion (12) of the first leg (10) is connectable to a patient resting structure (200);
wherein the second end portion (14) of the first leg (10) is connected to the first end portion (52) of the yoke structure (50);
wherein at least a first one of the intermediate portion (16) of the first leg (10) and the intermediate portion (56) of the yoke (50) comprises a plurality of carbon fibers (80) being embedded in a non-conductive matrix (90), wherein the plurality of carbon fibers (80) in the respective intermediate portion (16, 56) are isolated from each other so as to avoid conductive loops.

2. MRI compatible frame according to claim 1, further comprising:
a second leg (30) having a first end portion (32), a second end portion (34) and an intermediate portion (36) longitudinally extending between the first and second end portion of the second leg;
wherein the first end portion (32) of the second leg structure (30) is connectable to a patient resting structure (200);
wherein the second end portion (34) of the second leg structure (30) is connected to the second end portion (54) of the yoke (50);
wherein at least a first one of the intermediate portion (16) of the first leg (10), the intermediate portion (36) of the second leg (30) and the intermediate portion (56) of the yoke (50) comprises a plurality of carbon fibers (80) being embedded in a non-conductive matrix (90), wherein the plurality of carbon fibers (80) in the respective intermediate portion (16, 36, 56) are isolated from each other so as to avoid conductive loops.

3. MRI compatible frame according to any one of claims 1 and 2, wherein the plurality of carbon fibers (80) in the respective intermediate portion (16, 56) are substantially parallel to each other so as to avoid conductive loops.

4. MRI compatible frame according to any one of claims 1 to 3, further comprising a mouthpiece (70) and a mouthpiece lever (75), wherein the mouthpiece lever (75) connects the mouthpiece (70) and the yoke (50), wherein the mouthpiece lever is adaptable to bring the mouthpiece to an appropriate position and orientation with respect to a patient (1) resting on the patient resting structure (200) such that the patient can be brought into a defined position with respect to the MRI compatible frame (100).

5. MRI compatible frame according to any one of claims 1 to 4, wherein at least a second one of the intermediate portion (16) of the first leg (10), the intermediate portion (36) of the second leg (30) and the intermediate portion (56) of the yoke (50) comprises a plurality of carbon fibers (80) being embedded in a non-conductive matrix (90), wherein the plurality of carbon fibers (80) in the respective intermediate section are isolated from each other so as to avoid conductive loops;
wherein at least one of the end portions (14, 34, 52, 54) of the first leg (10), the second leg (30) and/or the yoke (50) being connected to another one of the end portions (14, 34, 52, 54) of the first leg (10), the second leg (30) and/or the yoke (50) is adapted to avoid an electrically conducting connection between the carbon fibers (80) of the first leg (10), the second leg (30) and the yoke (50), and the carbon fibers (80) of a connected one of the first leg (10), the second leg (30) and the yoke (50).

6. MRI compatible frame according to any one of claims 1 to 5, wherein the intermediate portion (16, 36) of at least one of the first leg (10) and the second leg (30) follow an offset Z- or S-shape, wherein a section (17, 37) of the intermediate portion (16, 36) close to the first end (12, 32) of the respective leg (10, 30) and a section (18, 38) of the intermediate portion (16, 36) close to the second end (14, 34) of the respective leg (10, 30) are substantially parallel.

7. MRI compatible frame according to any one of claims 1 to 6, wherein at least one of the intermediate portions (16, 36, 56) of the first leg (10), the second leg (30) and the yoke (50) is arc shaped, wherein the carbon fibers (80) in the respective intermediate portion follow the respective arc shape.

8. MRI compatible frame according to claim 7, wherein each of the intermediate portions (16, 36, 56) of the first leg (10), the second leg (30) and the yoke (56) are arc shaped, wherein the first leg (10), the second leg (30) and the yoke (50) together form a substantially continuously bended arc.

9. MRI compatible frame according to any one of claims 1 to 8, wherein at least one of the first leg (10), the second leg (30) and the yoke (50) comprises at least two separated and isolated longitudinal sub-sections (21, 22; 41, 42; 61, 62) being aligned in a longitudinal direction of the respective intermediate portion (16, 36, 56), wherein each of the longitudinal sub-sections (21, 22; 41, 42; 61, 62) comprises a plurality of carbon fibers (80) being embedded in a non-conductive matrix (90), wherein the plurality of carbon fibers (80) in the respective longitudinal sub-section (21, 22; 41, 42; 61, 62) are isolated from each other so as to avoid conductive loops, wherein the sub-sections of a respective first leg (10), second leg (30) and/or yoke (50) are electrically isolated with respect to each other.

10. MRI compatible frame according to any one of claims 1 to 9, wherein at least one of the first leg (10), the second leg (30) and the yoke (50) comprises at least two separated and isolated lateral sub-sections (21, 23; 41, 43; 61, 63) laterally aligned with respect to a longitudinal direction of the respective intermediate portion (16, 36, 56), wherein each of the lateral sub-sections (21, 23; 41, 43; 61, 63) comprises a plurality of carbon fibers (80) being embedded in a non-conductive matrix (90), wherein the plurality of carbon fibers (80) in the respective lateral sub-section (21, 23; 41, 43; 61, 63) are isolated from each other so as to avoid conductive loops, wherein the sub-sections of a respective first leg (10), second leg (30) and/or yoke (50) are electrically isolated with respect to each other.

11. MRI compatible frame according to any one of claims 1 to 10, wherein at least one of the end portions (12, 14, 32, 34, 52, 54) of the first leg (10), the second leg (30) and the yoke (50) comprise a coupling member (11, 13, 31, 33, 51, 53) matching a cooperating coupling member (11, 13, 31, 33, 51, 53) of another one of the end portions (12, 14, 32, 34, 52, 54) of the first leg (10), the second leg (30) and the yoke (50) coupled thereto, wherein the matching coupling members can be brought from a locked state into a releasable state and vice versa.

12. MRI compatible frame according to claim 11, wherein the coupling member (11, 13, 31, 33, 51, 53) comprises an arrangement of pins and/or holes matching an arrangement of pins and/or holes of a cooperating coupling member (11, 13, 31, 33, 51, 53) which is intended to be connected to the coupling member.

13. MRI compatible frame according to claim 12, wherein at least one of the end portions (12, 14, 32, 34, 52, 54) of the first leg (10), the second leg (30) and the yoke (50) comprise a fast lock connector (99) being adapted to bring the matching coupling members (11, 13, 31, 33, 51, 53) from a released state into a locked state by applying a pressing force in a direction which corresponds to an insertion direction of one of the pins into one of the holes.

14. MRI compatible frame according to any one of claims 1 to 12, wherein the first leg (10), the second leg (30) and the yoke (50) are adhesively connected to each other, wherein an adhesive agent forms an electrical isolation between the first leg (10) and the yoke (50) as well as the second leg (30) and the yoke (50).

15. MRI compatible frame according to any one of claims 1 to 14, wherein the entirety of the first leg (10), the second leg (30) and the yoke (50) is provided with an arrangement of markers (98) such that the spatial orientation of the MRI compatible frame is uniquely recognizable in a fluoroscopic projection or MR imaging.

16. MRI compatible frame according to any one of claims 1 to 15, wherein the first leg (10), the second leg (30) and the yoke (50) at least partially are manufactured from an insulating material which has a B0 distortion of a homogeneous B0 magnetic field of less than 0.25 ppm inside a relevant MR imaging volume so as to not generate unacceptable distortion in MR images.
